# EUROPEAN PATENT SPECIFICATION

(11) **EP 2 967 640 B1**
(45) Date of publication and mention of the grant of the patent: **08.04.2020**
(21) Application number: 14776381.7
(22) Date of filing: 14.02.2014
(51) Int. Cl.: A61B 17/3207

(54) **DEVICES FOR A PILOTING TIP BUSHING FOR ROTATIONAL ATHERECTOMY**
VORRICHTUNGEN ZUM ANSTEUERN EINER BUCHSENSPITZE FÜR EINE ATHEREKTOMIE
DISPOSITIFS POUR UNE BAGUE DE POINTE DE PILOTAGE EMPLOYÉE POUR UNE ATHÉRECTOMIE ROTATIVE

(30) Priority: 14.03.2013 US 201361782083 P; 28.01.2014 US 201414166207
(43) Date of publication of application: 20.01.2016
(73) Proprietor: Cardiovascular Systems, Inc., New Brighton, Minnesota 55112 (US)
(72) Inventor: ELLERING, Nicholas, Crystal, Minnesota 55429 (US); HIGGINS, Joseph, Minnetonka, Minnesota 55305 (US)
(74) Representative: Viering, Jentschura & Partner mbB Patent- und Rechtsanwälte
(86) International application number: PCT/US2014/016350
(87) International publication number: WO 2014/158416

(56) References cited:
- US-A- 4 696 667
- US-A- 5 843 103
- US-A1- 2001 004 700
- US-A1- 2003 055 444
- US-A1- 2009 105 736
- US-A1- 2009 264 908
- US-A1- 2009 306 690
- US-A1- 2009 306 690
- US-B1- 6 482 216

## Description

### CROSS-REFERENCE TO RELATED APPLICATIONS

This application claims priority to App. Ser. No. 61/782,083, entitled "Devices, Systems and Methods for a Piloting Tip Bushing for Rotational Atherectomy," filed March 14, 2013.

### FIELD OF THE INVENTION

The invention relates to devices for removing tissue from body passageways, such as removal of atherosclerotic plaque from arteries, utilizing a high-speed rotational atherectomy device.

### DESCRIPTION OF THE RELATED ART

A variety of techniques and instruments have been developed for use in the removal or repair of tissue in arteries and similar body passageways. A frequent objective of such techniques and instruments is the removal of atherosclerotic plaques in a patient's arteries. Atherosclerosis is characterized by the buildup of fatty deposits (atheromas) in the intimal layer (under the endothelium) of a patient's blood vessels. Very often over time, what initially is deposited as relatively soft, cholesterol-rich atheromatous material hardens into a calcified atherosclerotic plaque. Such atheromas restrict the flow of blood, and therefore often are referred to as stenotic lesions or stenoses, the blocking material being referred to as stenotic material. If left untreated, such stenoses can cause angina, hypertension, myocardial infarction, strokes and the like.

Rotational atherectomy procedures have become a common technique for removing such stenotic material. Such procedures are used most frequently to initiate the opening of calcified lesions in coronary arteries. Most often the rotational atherectomy procedure is not used alone, but is followed by a balloon angioplasty procedure, which, in turn, is very frequently followed by placement of a stent to assist in maintaining patentcy of the opened artery. For non-calcified lesions, balloon angioplasty most often is used alone to open the artery, and stents often are placed to maintain patentcy of the opened artery. Studies have shown, however, that a significant percentage of patients who have undergone balloon angioplasty and had a stent placed in an artery experience stent restenosis-i.e., blockage of the stent which most frequently develops over a period of time as a result of excessive growth of scar tissue within the stent. In such situations an atherectomy procedure is the preferred procedure to remove the excessive scar tissue from the stent (balloon angioplasty being not very effective within the stent), thereby restoring the patentcy of the artery.

Several kinds of rotational atherectomy devices have been developed for attempting to remove stenotic material. In one type of device, such as that shown in U.S. Pat. No. 4,990,134 (Auth), a burr covered with an abrasive abrading material such as diamond particles is carried at the distal end of a flexible drive shaft. The burr is rotated at high speeds (typically, e.g., in the range of about 150,000-190,000 rpm) while it is advanced across the stenosis. As the burr is removing stenotic tissue, however, it blocks blood flow. Once the burr has been advanced across the stenosis, the artery will have been opened to a diameter equal to or only slightly larger than the maximum outer diameter of the burr. Frequently more than one size burr must be utilized to open an artery to the desired diameter.

U.S. Pat. No. 5,314,438 (Shturman) discloses another atherectomy device having a drive shaft with a section of the drive shaft having an enlarged diameter, at least a segment of this enlarged surface being covered with an abrasive material to define an abrasive segment of the drive shaft. When rotated at high speeds, the abrasive segment is capable of removing stenotic tissue from an artery. Though this atherectomy device possesses certain advantages over the Auth device due to its flexibility, it also is capable only of opening an artery to a diameter about equal to the diameter of the enlarged abrading surface of the drive shaft since the device is not eccentric in nature.

U.S. Pat. No. 6,494,890 (Shturman) discloses a known atherectomy device having a drive shaft with an enlarged eccentric section, wherein at least a segment of this enlarged section is covered with an abrasive material. When rotated at high speeds, the abrasive segment is capable of removing stenotic tissue from an artery. The device is capable of opening an artery to a diameter that is larger than the resting diameter of the enlarged eccentric section due, in part, to the orbital rotational motion during high speed operation. Since the enlarged eccentric section comprises drive shaft wires that are not bound together, the enlarged eccentric section of the drive shaft may flex during placement within the stenosis or during high speed operation. This flexion allows for a larger diameter opening during high speed operation, but may also provide less control than desired over the diameter of the artery actually abraded. In addition, some stenotic tissue may block the passageway so completely that the Shturman device cannot be placed therethrough. Since Shturman requires that the enlarged eccentric section of the drive shaft be placed within the stenotic tissue to achieve abrasion, it will be less effective in cases where the enlarged eccentric section is prevented from moving into the stenosis.

U.S. Pat No. 5,681, 336 (Clement) provides a known eccentric tissue removing burr with a coating of abrasive particles secured to a portion of its outer surface by a suitable binding material. This construction is limited, however because, as Clement explains at CoI. 3, lines 53-55, that the asymmetrical burr is rotated at "lower speeds than are used with high speed ablation devices, to compensate for heat or imbalance." That is, given both the size and mass of the solid burr, it is infeasible to rotate the burr at the high speeds used during atherectomy procedures, i.e., 20,000-200,000 rpm. Essentially, the center of mass offset from the rotational axis of the drive shaft would result in development of significant centrifugal force, exerting too much pressure on the wall of the artery and creating too much heat and excessively large particles.

In some situations, at the high rotational speeds of the atherectomy device, when the device is driven into the lesion, it can screw into the lesion. Moreover, the atherectomy device may be limited to a certain size of lesion or stenosis for treatment because of the diameter of the burr. For these and other reasons, it may be desirable to include an abrasive structure positioned distally from the ablation burr to first create a piloting hole in the stenosis before the abrading head contacts the stenosis. Prior art devices, such as U.S. Patent No. 6,482,216 (Hiblar), have suggested a concentric ablation burr mounted on the driveshaft and a concentric abrasive tip mounted on the end of the guidewire to ablate deposits from the blood vessel or stent without becoming embedded in the deposits as the abrasive tip engages the deposits. However, with such devices, the path and diameter of treatment is limited to the minimum size lesion. Prior art U.S. Patent Nos. 5,843,103 (Wulfman) and 2009/0306690 (Rivers) disclose further examples of devices known in the field.

The present invention overcomes these deficiencies and provides, inter alia, the above-referenced improvements.

### BRIEF SUMMARY OF THE INVENTION

The present system is directed to a device relating to rotational atherectomy. More specifically, a piloting tip is mounted distal to an abrasive element, the piloting tip or bushing comprising a shape and structure to facilitate opening pilot holes through difficult occlusions and/or stenosis.

The high-speed rotational atherectomy device for opening a stenosis in an artery having a given diameter, comprises a guide wire having a maximum diameter less than the diameter of the artery; a flexible elongated, rotatable drive shaft advanceable over the guide wire, the drive shaft having a proximal end and a distal end; an abrasive section; and a piloting member fixedly attached to the drive shaft and disposed distally of the abrasive section. The abrasive section has an eccentric abrading head. The piloting member has a concentric profile.

In at least one embodiment, a piloting member or piloting tip comprises a proximal section extending distally from a proximal end of the piloting member, the proximal section having a constant diameter; a distal section extending proximally from a distal end of the piloting member having a diameter at the distal end less than a diameter at the proximal end of the piloting member, the diameter increasing proximally from the distal end; and an intermediate section between the proximal section and the distal section, the intermediate section having a generally parabolic profile, wherein the diameter of the piloting member increases from the constant diameter of the proximal section to a maximum point and then decreases distally towards the distal section. The piloting tip is concentric. In some embodiments, the piloting tip has an inner lumen at least at the proximal section with a diameter greater than the diameter of the drive shaft. In at least one embodiment, the piloting tip has a diameter less than a diameter of the drive shaft.

A exemplary method not forming part of the invention, for opening a stenosis in a blood vessel having a given diameter is also provided, the method comprising: providing a guide wire having a maximum diameter less than the diameter of the artery; advancing the guide wire into a blood vessel to a position proximal to the stenosis; providing a flexible elongated, rotatable drive shaft advanceable over a guide wire, the guide wire having a maximum diameter less than the diameter of the artery; the drive shaft having a rotational axis; the drive shaft having at least one eccentric abrading head and a piloting tip fixedly attached to the drive shaft and disposed distally from the abrading head; advancing the piloting tip into the artery to a position proximal to the stenosis; creating a piloting hole by rotating the drive shaft at a sufficient rotational speed; advancing the eccentric abrading head through the piloting hole, rotating the drive shaft at the rotational speed, and advancing the eccentric abrading head distally across the stenotic lesion, thereby opening the stenotic lesion to a diameter larger than the nominal diameter of the eccentric enlarged diameter section. In some embodiments, the piloting tip has an orbital path such that the piloting hole has a diameter greater than a maximum diameter of the piloting tip. In some embodiments, an axial distance between a proximal end of the piloting tip and the abrading head remains constant.

### BRIEF DESCRIPTION OF THE DRAWINGS

FIG. 1 is a perspective view of a rotational atherectomy device of the invention.
FIG. 2 is a perspective view of an exemplary piloting tip of the invention.
FIG. 3 is a side view of the exemplary piloting tip of FIG. 2.
FIG. 4 is a view of the exemplary piloting tip of FIGS. 2-3 from a distal end of the tip.
FIG. 5 is a view of the exemplary piloting tip of FIGS. 2-4 from a proximal end of the tip.
FIG. 6 is a perspective view of an exemplary piloting tip of the invention.
FIG. 7 is a side view of the exemplary piloting tip of FIG. 6.
FIG. 8 is a view of the exemplary piloting tip of FIGS. 6-7 from a distal end of the tip.
FIG. 9 is a view of the exemplary piloting tip of FIGS. 6-8 from a proximal end of the tip.

### DETAILED DESCRIPTION

While the invention is amenable to various modifications and alternative forms, specifics thereof are shown by way of example in the drawings and described in detail herein. It should be understood, however, that the intention is not to limit the invention to the particular embodiments described. On the contrary, the intention is to cover all modifications, equivalents, and alternatives falling within the scope of the invention, as defined by the appended claims.

Various embodiments of the present invention comprise a rotational atherectomy system as described generally in US 6,494,890, entitled "ECCENTRIC ROTATIONAL ATHERECTOMY DEVICE,".

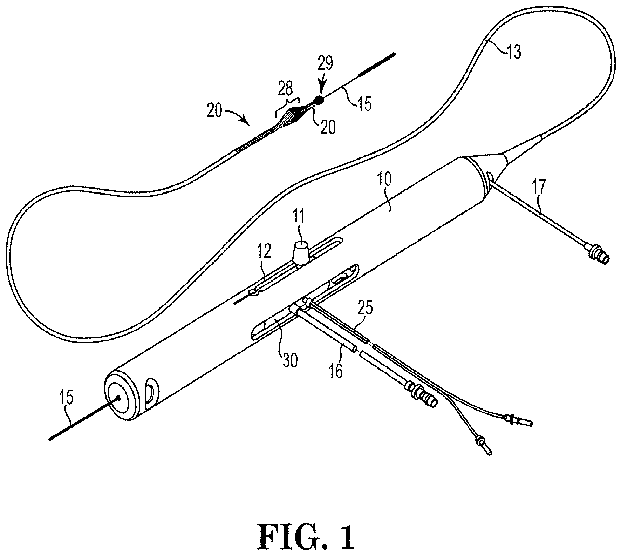

FIG. 1 illustrates one embodiment of a rotational atherectomy device according to the present invention. The device includes a handle portion 10; an elongated, flexible drive shaft 20 having an eccentric abrading head 28 and a piloting section comprising either a piloting tip or bushing 29 mounted or otherwise disposed on the flexible drive shaft at a point distal to the abrading head 28; and an elongated catheter 13 extending distally from the handle portion 10. The drive shaft 20 is constructed from helically coiled wire as is known in the art and the abrading head 28 and the piloting tip or bushing 29 are fixedly attached to the drive shaft 20. The drive shaft 20 has an outer surface 24 and an inner surface 22 defining an inner lumen, permitting the drive shaft 20 to be advanced and rotated over a guide wire 15. The catheter 13 has a lumen in which most of the length of the drive shaft 20 is disposed, except for the enlarged abrading head 28 and a section of the drive shaft 20 distal to the enlarged abrading head 28. A fluid supply line 17 may be provided for introducing a cooling and lubricating solution (typically saline or another biocompatible fluid) into the catheter 13.

The handle 10 desirably contains a turbine (or similar rotational drive mechanism) for rotating the drive shaft 20 at high speeds. The handle 10 typically may be connected to a power source, such as compressed air delivered through a tube 16. A pair of fiber optic cables 25, alternatively a single fiber optic cable may be used, may also be provided for monitoring the speed of rotation of the turbine and drive shaft 20 (details regarding such handles and associated instrumentation are well known in the industry, and are described, e.g., in U.S. Pat. No. 5,314,407, issued to Auth). The handle 10 also desirably includes a control knob 11 for advancing and retracting the turbine and drive shaft 20 with respect to the catheter 13 and the body of the handle.

As discussed above, in at least one embodiment, the eccentric abrading head 28 comprises an eccentric enlarged section of the drive shaft, or an eccentric solid crown, or an eccentric burr attached to the drive shaft. In some embodiments, the abrasive section 28 has a center of mass spaced radially from the rotational axis of the drive shaft 20, facilitating the ability of the device to open the stenotic lesion to a diameter substantially larger than the outer diameter of the abrasive section 28. This may be achieved by spacing the geometric center of the abrasive section 28, i.e., the eccentric enlarged diameter section of the drive shaft 20, or the eccentric solid abrading head or crown, or burr attached to the drive shaft 20, away from the rotational axis of the drive shaft 20. Alternatively, the center of mass of the abrading head 28 may be radially spaced from the drive shaft's rotational axis by providing an abrading head 28 that comprises a differential combination of materials, wherein one side of at least one of the abrading head 28 comprises a more massive or denser material than the other side, which creates eccentricity as defined herein. As those skilled in the art will recognize, creation of eccentricity as by differential use of materials within the structure of the abrading head 28, e.g., a center of mass offset from the drive shaft's rotational axis, is applicable to any embodiment of the abrading head 28 discussed herein, whether concentric, eccentric solid burr, partially hollow crown or abrading head or an enlarged section of the drive shaft, or the equivalent. When rotated at high rotational speeds, the drive shaft 20 stimulates orbital motion of the eccentric abrading head 28 to generate a cutting diameter that is greater than a diameter of the abrading head.

In the present invention, the abrading head 28 comprises an eccentric profile. In some embodiments, the abrading head 28 may achieve orbital motion, generated by a positioning of the center of mass of the abrading head 28 radially offset from the rotational axis of the drive shaft, either by using different densities of materials and/or geometrically moving the center of mass of the abrading head 28 radially away from the drive shaft's center of mass. This "eccentricity" may be achieved in either a concentric or an eccentric geometric profile. The abrading head 28 may be an enlarged section of the drive shaft, a burr, or a contoured abrasive element and may comprise diamond coating. In other embodiments, the abrading head 28 may comprise a center of mass that is on the drive shaft's rotational axis.

However, these known abrading heads 28 described above are limited to the minimum size lesions that can be treated because the abrasive features of the abrasive element are of a diameter that is larger than the drive shaft diameter. The present device remedies that problem, among others. Further, if known abrasive elements are forced or driven into a lesion, the abrading head 28 may grip and screw/auger into the lesion with a subsequent building and releasing of force that may undesirably affect the lesion or the blood vessel. The present invention addresses this problem by opening a pilot hole with a diameter equivalent to the diameter of the flexible drive shaft of the atherectomy system. This allows for the minimum required clearance between the abrading head and the lesion to prevent gripping and screwing into the lesion.

The piloting tip or bushing 29 may be fixedly attached to the drive shaft 20, either by being mounted directly onto the outer surface of the drive shaft or mounted axially to the drive shaft at a distal end of the drive shaft. Since the piloting tip or bushing 29 is fixedly attached to the drive shaft 20, where the abrading head is also fixedly attached, the piloting tip or bushing 29 will rotate in the same direction and at the same speed as the abrading head 28.

The piloting tip or bushing 29 may be coupled with an eccentric profile abrading head 28 as discussed above. The piloting tip or bushing 29 may be coupled with an abrading head 28 of an eccentric geometric profile, wherein the abrading head's center of mass is offset radially from the drive shaft's center of mass. The piloting tip or bushing 29 may also be coupled with an abrading head 28 of eccentric geometric profile, wherein the abrading head's enter of mass is collinear with the drive shaft's center of mass. The piloting tip or bushing 29 comprises a concentric profile. The piloting tip or bushing may also comprise a center of mass that is either collinear with the rotational axis of the drive shaft or that is offset radially from the drive shaft's rotational axis using the same techniques discussed above in connection with the abrasive element. In at least one embodiment, where the abrading head is eccentric and the piloting tip or bushing is concentric, the abrading head will act as a counterweight, causing orbital motion of the piloting tip and thereby creating an increased rotational diameter for the abrading head. In some examples not forming part of the invention, the abrading head and the piloting tip are both eccentric and in still other examples, the abrading head and the piloting tip are both concentric.

The piloting tip or bushing 29 may be spaced apart from the abrading head 28 along the drive shaft 20. In other embodiments, a proximal end of the piloting tip or bushing abuts a distal end of the abrading head 28. Piloting tip or bushing 29 in at least some embodiments comprises a distalmost tip that is of the same diameter as the drive shaft to facilitate opening of stenosis in preparation for the abrasive element's rotational entry therein.

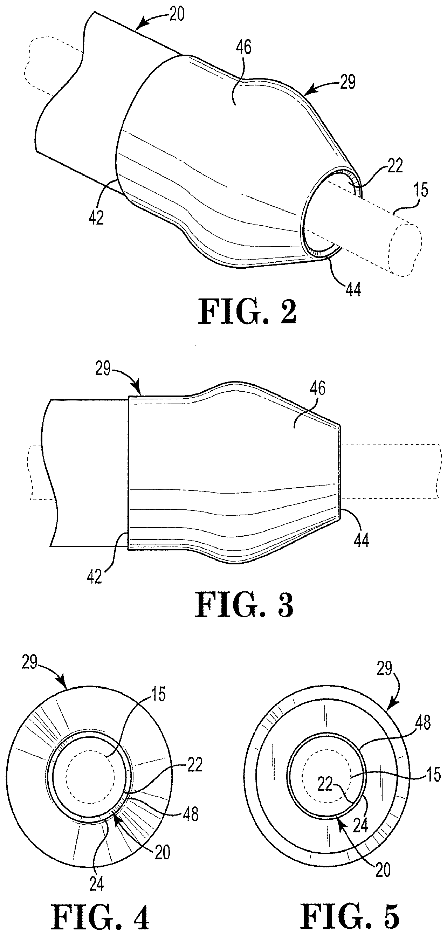

The piloting tip or bushing 29 may have profiles as illustrated in FIGS. 2-9. The piloting tip or bushing 29 has a proximal end 42, a distal end 44, an outer surface 46, and an inner surface 48 that defines a lumen. In some embodiments, where the piloting tip or bushing 29 is fixedly disposed about an outer surface of the drive shaft 20, the inner surface 48 of the piloting tip or bushing 29 mates or is engaged with the outer surface 24 of the drive shaft 20. In other embodiments, the piloting tip or bushing 29 may be fixedly attached to a distal end of the drive shaft 20, and the lumen defined by the inner surface 48 allows the piloting tip or bushing 29 to be advanced and rotated over a guide wire 15. Importantly, the piloting tip or bushing 29 is fixedly disposed to the outer surface of the drive shaft or fixedly attached to a distal end of the drive shaft 20 such that it rotates simultaneously with the abrading head, rather than separately or selectively rotated.

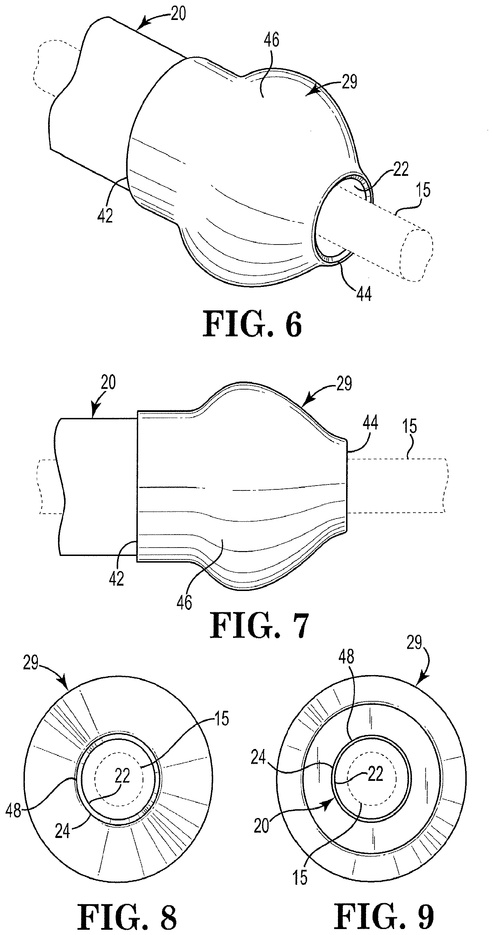

The piloting tip or bushing 29 may have a shape with a distal end having a diameter smaller than the proximal end. In some embodiments, the piloting tip increases in diameter from the distal end 44 to the proximal end 42. In some embodiments, the piloting tip has a bulbous profile. In some embodiments, such as the embodiments shown in FIGS. 2-3, the outer diameter of the piloting tip or bushing 29 has a constant diameter in a proximal section extending distally of the proximal end 42; in an intermediate section, the diameter of the piloting tip or bushing 29 increases to a maximum point at a distal end of the intermediate section; and in a distal section, the diameter of the piloting tip or bushing tapers at a constant slope to a diameter at the distal end 44 less than the constant diameter at the proximal end. In some embodiments, such as the embodiment shown in FIGS. 6-7, the outer diameter of the piloting tip or bushing 29 has a constant diameter in a proximal section extending distally of the proximal end 42; in an intermediate section, the diameter of the piloting tip or bushing 29 increases to a maximum point at a distal end of the intermediate section; and in a distal section, the outer diameter of the piloting tip or bushing decreases to a diameter at the distal end 44 less than the constant diameter at the proximal end. In some embodiments, the outer diameter of the piloting tip may decrease to a diameter less than the outer diameter of the drive shaft. In the embodiments shown in FIGS. 2-9, the piloting tip is symmetrical about a central axis. In other embodiments, the piloting tip is asymmetrical about the central axis, such that the piloting tip has an orbital path, which may or may not be different than the orbital path of the abrading head.

The piloting tip or bushing 29 may have an abrasive coating disposed on some or all of the outer surface 46 of the piloting tip or bushing 29. The abrasive coating may be disposed in discrete areas in a desired pattern. In some embodiments, the piloting tip or bushing 29 has a cutting feature on the outer surface 46. In some embodiments, the piloting tip or bushing 29 has an impact feature on the outer surface 46. In some embodiments, the piloting tip or bushing 29 has a thread-like cutting feature disposed about the outer surface 46. In some embodiments, the piloting tip or bushing 29 is shaped like an auger drill bit with a helical screw blade.

An exemplary method for opening a stenosis in a blood vessel having a given diameter, comprising: providing a guide wire having a maximum diameter less than the diameter of the artery; advancing the guide wire into a blood vessel to a position proximal to the stenosis; providing a flexible elongated, rotatable drive shaft advanceable over a guide wire, the guide wire having a maximum diameter less than the diameter of the artery; the drive shaft having a rotational axis; the drive shaft having at least one eccentric abrading head and a piloting tip fixedly attached to the drive shaft; advancing the piloting tip into the artery to a position proximal to the stenosis; creating a piloting hole by rotating the drive shaft at a sufficient rotational speed; advancing the eccentric abrading head through the piloting hole, rotating the drive shaft at the rotational speed, and moving the across the stenotic lesion, thereby opening the stenotic lesion to a diameter larger than the nominal diameter of the eccentric enlarged diameter section.

Any and all of the above combinations of piloting tip or bushing and the abrasive element in rotational atherectomy system are within the scope of the present invention, provided they fall within the scope as defined by the appended claims. The present invention should not be considered limited to the particular examples described above, but rather should be understood to cover all aspects of the invention. Various modifications, equivalent processes, as well as numerous structures to which the present invention may be applicable will be readily apparent to those of skill in the art to which the present invention is directed upon review of the present specification.

## Claims

1. A high-speed rotational atherectomy device for opening a stenosis in an artery having a given diameter, comprising:
a guide wire (15) having a maximum diameter less than the diameter of the artery;
a flexible elongated, rotatable drive shaft (20) advanceable over the guide wire, the drive shaft having a proximal end and a distal end and an axis of rotation;
an eccentric abrading head (28) attached proximate to the distal end of the drive shaft and comprising a center of mass that is radially offset from the axis of rotation of the drive shaft; and
a concentric piloting member (29) fixedly attached to the distal end of the drive shaft and disposed distal to and spaced apart from the eccentric abrading head, wherein the concentric piloting member has an outer surface (46) with an abrasive coating on at least a portion of the piloting member and a center of mass located on the axis of rotation of the drive shaft.

2. The high-speed rotational atherectomy device of claim 1, wherein the piloting member (29) is mounted onto the outer surface (24) of the drive shaft (20).

3. The high-speed rotational atherectomy device of claim 1, wherein the piloting member (29) is mounted axially to the drive shaft (20) at a distal end of the drive shaft.

4. The high-speed rotational atherectomy device of claim 1, wherein the piloting member (29) has a bulbous profile.

5. The high-speed rotational atherectomy device of claim 1, wherein the piloting member (29) comprises:
a proximal section extending distally from a proximal end (42) of the piloting member, the proximal section having a constant diameter;
a distal section extending proximally from a distal end (44) of the piloting member having a diameter at the distal end (44) less than a diameter at the proximal end (42) of the piloting member, the diameter increasing proximally from the distal end (44);
an intermediate section between the proximal section and the distal section, the intermediate section having a generally parabolic profile, wherein the diameter of the piloting member increases from the constant diameter of the proximal section to a maximum point and then decreases distally towards the distal section.

6. The high-speed rotational atherectomy device of claim 6, wherein the diameter of the distal section increases proximally from the distal end (44) at a constant slope.

7. The high-speed rotational atherectomy device of claim 6, wherein the diameter of the distal section increases parabolically from the distal end (44) towards a proximal end (42).

## Patentansprüche

1. Vorrichtung zur Hochgeschwindigkeits-Rotationsatherektomie zum Öffnen einer Stenose einer Arterie, die einen gegebenen Durchmesser aufweist, die Vorrichtung aufweisend:
einen Führungsdraht (15), der einen maximalen Durchmesser, der kleiner ist als der Durchmesser der Arterie, aufweist;
eine flexible, längliche, rotierende Antriebswelle (20), die über den Führungsdraht (15) bewegt werden kann, wobei die Antriebswelle (20) ein proximales Ende und ein distales Ende und eine Rotationsachse (21) aufweist; und
einen exzentrischen Abschleifkopf (28), der nahe zu dem distalen Ende der Antriebswelle angebracht ist, und der einen Massemittelpunkt, der von der Rotationsachse der Antriebswelle radial versetzt ist, aufweist; und
ein konzentrisches Ansteuerelement (29), das an dem distalen Ende der Antriebswelle fest angebracht ist, und das distal zu und in einem Abstand von dem exzentrischen Abschleifkopf angeordnet ist, wobei das konzentrische Ansteuerelement eine Außenfläche (46) mit einer Abriebbeschichtung auf zumindest einem Abschnitt des Ansteuerelementes und einen Massemittelpunkt, der sich auf der Rotationsachse der Antriebswelle befindet, aufweist.

2. Vorrichtung zur Hochgeschwindigkeits-Rotationsatherektomie nach Anspruch 1, wobei das Ansteuerelement (29) an der Außenfläche (24) der Antriebswelle (20) angebracht ist.

3. Vorrichtung zur Hochgeschwindigkeits-Rotationsatherektomie nach Anspruch 1, wobei das Ansteuerelement (29) axial zu der Antriebswelle (20) an einem distalen Ende der Antriebswelle angebracht ist.

4. Vorrichtung zur Hochgeschwindigkeits-Rotationsatherektomie nach Anspruch 1, wobei das Ansteuerelement (29) ein bauchiges Profil aufweist.

5. Vorrichtung zur Hochgeschwindigkeits-Rotationsatherektomie nach Anspruch 1, wobei das Ansteuerelement (29) aufweist:
einen proximalen Abschnitt, der sich distal von einem proximalen Ende (42) des Ansteuerelements erstreckt, wobei der proximale Abschnitt einen konstanten Durchmesser aufweist;
einen distalen Abschnitt, der sich proximal von einem distalen Ende (44) des Ansteuerelements erstreckt, aufweisend einen Durchmesser an dem distalen Ende (44), der geringer ist als ein Durchmesser an dem proximalen Ende (42) des Ansteuerelements, wobei der Durchmesser von dem distalen Ende (44) proximal größer wird;
einen mittleren Abschnitt zwischen dem proximalen Abschnitt und dem distalen Abschnitt, wobei der mittlere Abschnitt ein im Wesentlichen parabolisches Profil aufweist, wobei der Durchmesser des Ansteuerelements von dem konstanten Durchmesser des proximalen Abschnitts zu einem Maximalpunkt größer wird und danach distal in Richtung des distalen Abschnitts kleiner wird.

6. Vorrichtung zur Hochgeschwindigkeits-Rotationsatherektomie nach Anspruch 6, wobei der Durchmesser des distalen Abschnitts von dem distalen Ende (44) proximal mit konstanter Neigung größer wird.

7. Vorrichtung zur Hochgeschwindigkeits-Rotationsatherektomie nach Anspruch 6, wobei der Durchmesser des distalen Abschnitts von dem distalen Ende (44) in Richtung eines proximalen Endes (42) parabolisch größer wird.

## Revendications

1. Dispositif d'athérectomie rotatif à vitesse élevée destiné à ouvrir une sténose dans une artère qui présente un diamètre donné, comprenant :
un fil guide (15) qui présente un diamètre maximum inférieur au diamètre de l'artère ;
une tige d'entraînement rotative, allongée et souple (20) qui peut avancer sur le fil guide, la tige d'entraînement présentant une extrémité proximale, une extrémité distale et un axe de rotation ;
une tête abrasive excentrique (28) fixée à proximité de l'extrémité distale de la tige d'entraînement, et comprenant un centre de gravité qui est décalé radialement de l'axe de rotation de la tige d'entraînement ; et
un élément de pilotage concentrique (29) fixé de manière fixe sur l'extrémité distale de la tige d'entraînement, et disposé de manière distale par rapport à la tête abrasive excentrique en étant espacé de celle-ci, où l'élément de pilotage concentrique présente une surface extérieure (46) dotée d'un revêtement abrasif sur une partie au moins de l'élément de pilotage, et un centre de gravité situé sur l'axe de rotation de la tige d'entraînement.

2. Dispositif d'athérectomie rotatif à vitesse élevée selon la revendication 1, où l'élément de pilotage (29) est monté sur la surface extérieure (24) de la tige d'entraînement (20).

3. Dispositif d'athérectomie rotatif à vitesse élevée selon la revendication 1, où l'élément de pilotage (29) est monté axialement par rapport à la tige d'entraînement (20) au niveau d'une extrémité distale de la tige d'entraînement.

4. Dispositif d'athérectomie rotatif à vitesse élevée selon la revendication 1, où l'élément de pilotage (29) présente un profil en bulbe.

5. Dispositif d'athérectomie rotatif à vitesse élevée selon la revendication 1, où l'élément de pilotage (29) comprend :
une section proximale qui s'étend de manière distale à partir d'une extrémité proximale (42) de l'élément de pilotage, la section proximale présentant un diamètre constant ;
une section distale qui s'étend de manière proximale à partir d'une extrémité distale (44) de l'élément de pilotage, qui présente un diamètre au niveau de l'extrémité distale (44) inférieur au diamètre au niveau de l'extrémité proximale (42) de l'élément de pilotage,
le diamètre croissant de manière proximale à partir de l'extrémité distale (44) ;
une section intermédiaire entre la section proximale et la section distale, la section intermédiaire présentant un profil généralement parabolique, où le diamètre de l'élément de pilotage croît à partir du diamètre constant de la section proximale jusqu'à un point maximum, et diminue ensuite de manière distale vers la section distale.

6. Dispositif d'athérectomie rotatif à vitesse élevée selon la revendication 6, où le diamètre de la section distale croît de manière proximale à partir de l'extrémité distale (44) selon une pente constante.

7. Dispositif d'athérectomie rotatif à vitesse élevée selon la revendication 6, où le diamètre de la section distale croît de manière parabolique à partir de l'extrémité distale (44) vers une extrémité proximale (42).
